(19) Europäisches Patentamt
European Patent Office
Office européen des brevets

(11) Publication number: 0 307 177 A2

(12) **EUROPEAN PATENT APPLICATION**

(21) Application number: 88308257.0

(51) Int. Cl.4: C07H 17/08 , A61K 31/00

(22) Date of filing: 07.09.88

The title of the invention has been amended (Guidelines for Examination in the EPO, A-III, 7.3).

(30) Priority: 09.09.87 GB 8721166

(43) Date of publication of application: 15.03.89 Bulletin 89/11

(84) Designated Contracting States: BE CH DE FR GB IT LI NL

(71) Applicant: BEECHAM GROUP PLC
Beecham House Great West Road
Brentford Middlesex TW8 9BD(GB)

(72) Inventor: Brain, Edward George
Beecham Pharmaceuticals Brockham Park
Betchworth Surrey, RH3 7AJ(GB)

(74) Representative: Dayneswood, Trevor et al
Beecham Pharmaceuticals Great Burgh Yew Tree Bottom Road
Epsom Surrey KT18 5XQ(GB)

(54) **Erythromycin derivatives.**

(57) 11,12-Carbonate derivatives of certain erythromycin 6-ethers are novel, antibacterially active compounds which can be prepared by treatment of an erythromycin 6-ether with a reactive carbonyl compound.

EP 0 307 177 A2

## CHEMICAL COMPOUNDS

The present invention relates to novel chemical compounds, their preparation and their use, and in particular to a novel class of erythromycin derivatives. These compounds have antibacterial properties, in particular against Gram-positive bacteria but also against some Gram-negative bacteria, and they are therefore of use in the treatment of bacterial infections in humans and animals caused by a wide range of organisms.

Erythromycin was first described in US 2 653 899 (R.L. Bunch et al; Eli Lilly). The structure of erythromycins can be represented as follows:

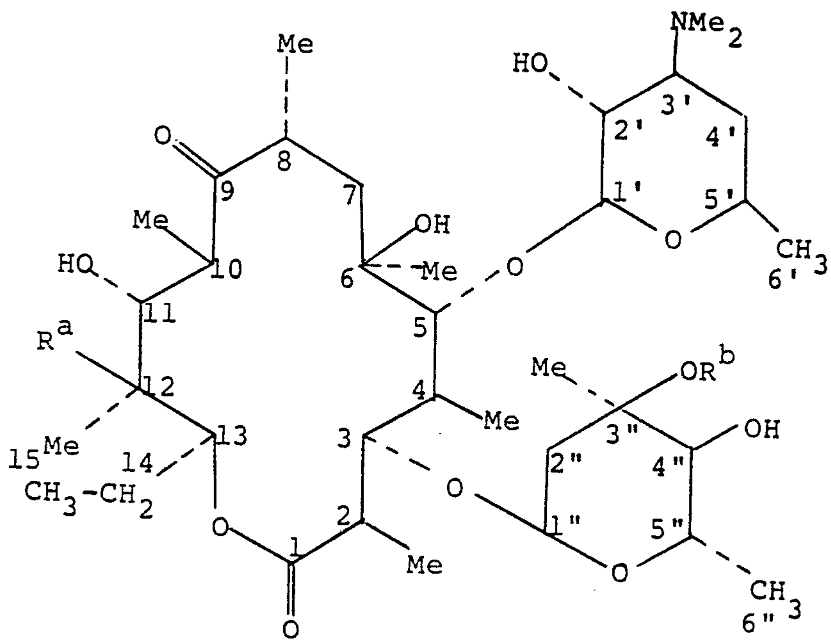

in which

$R^a$ denotes hydrogen or hydroxy and

$R^b$ denotes hydrogen or methyl.

The basic erythromycin structure comprises:

(i) a 14-membered lactone ring, referred to as the erythronolide ring, numbered with unprimed digits as shown in the above formula,

(ii) a first sugar ring, known as the desosamine ring, numbered with single-primed digits, and

(iii) a second sugar ring, known as the cladinose ring, numbered with double-primed digits.

The erythronolide ring can exist in two forms :

erythronoliode A (in which $R^a$ = OH)

erythronolide B (in which $R^a$ = H)

The four main naturally occurring erythromycins are as follows:

| Erythromycin | $R^a$ | $R^b$ |
|---|---|---|
| A | OH | $CH_3$ |
| B | H | $CH_3$ |
| C | OH | H |
| D | H | H |

of which erythromycin A is by far the most important.

Erythromycins, and in particular erythromycin A, are antibiotics widely employed clinically in the

treatment of infections caused by Gram-positive and some Gram-negative bacteria. A major drawback of erythromycins is their poor acid stability, resulting in poor and erratic oral absorption.

Numerous attempts have been made to modify erythromycin to produce derivatives having improved acid stability without loss of the antibacterial activity.

Among the many erythromycin derivatives that have been described in the literature is the 11,12-carbonate of erythromycin A, which is said to exist as the 6,9-hemiacetal in the solid state and to tautomerise to the 9-keto compound in certain solvents (W. Slawinski et al, Recueil, J. Royal Netherlands Chem. Soc. 94 236 (1975)). Other erythromycin 11,12-carbonate derivatives that have been described include the 11,12-carbonates of 9-dihydroerythromycin A (T. Glabski et al, Roczniki Chemii 50 1281 (1976)); of erythromycylamine (I. Dziegielewska et al, Polish J. Chem. 53 2551 (1979)); of 8-hydroxy-erythromycin A (K. Krowicki, J. Antibiotics XXVII (8) 626 (1974)); of erythromycin A L-aspartate salt (H. Bojarska-Dahlig et al, J. Antibiotics XXIX (9) 907 (1976)); of various 4″-modified erythromycin A derivatives (US 4 150 220, F.C. Scavolino, Pfizer; US 4 681 872, Freiberg et al, Abbott; US 4 686 207, Freiberg et al, Abbott (as the 6,9-hemiacetal form)); of erythromycylamine and various 2′-derivatives thereof (US 4 283 527, F.C.Sciavolino, Pfizer); of 2′-O,3′-N-bisbenzyloxycarbonyl-3′-N-desmethylerythromycin A 6,9-hemiacetal (JP 58.090 595, Taisho); and of 4″-O-methyl-erythromycin A 6,9-hemiacetal and 9-O,4″-O-dimethylerythromycin A 6,9-hemiacetal derivatives (EP 0 081 305, Taisho).

In addition, 11,12-carbonate derivatives of erythromycin have been described in which the 2′- or the 4″-hydroxyl group has been silylated (US 4 640 910, Faubl et al, Abbot), whilst 6,9-hemiacetal-8-9-anhydro-derivatives of erythromycin 11,12-carbonates have been described in which the 2′- or the 4″-position has been acylated or the substitutents on the 3′ - N varied (EP 0 215 355 and EP 0 213 617, Kitasto Kenkyusho).

All of the above 11,12-carbonate derivatives are prepared by the reaction of an appropriate erythromycin compound with ethylene carbonate in the presence of a base, and a modified method of carrying out that process has recently been described (EP 0 119 431, Polfa).

Another erythromycin derivative that has been described in the literature is 6-O-methylerythromycin A (EP 0 041 355, Taisho). That compound was obtained by treating 2′-O,3′-N-bisbenzyloxycarbonyl protected erythromycin A with methyl iodide in the presence of a strong base, followed by deprotection by hydrogenolysis and reductive methylation as described by E.H. Flynn et al, J. Amer. Chem. Soc., 1955, 77, 3104-31-6.

We were interested in preparing the 11,12-carbonate of 6-O-methylerythromycin A, which has not previously been described. Previous work (EP 0 081 305, Taisho, op cit.) has shown that methylation of 2′-O,3′-N-protected erythromycin A 11, 12-carbonate (with, for example, methyl iodine) results in methylation primarily at the 4″-position and optionally also at the 9-position of the 6,9-hemiacetal, to give, after deprotection, 4″-O-methylerythromycin A 6,9-hemiacetal 11,12-carbonate and 9-O,4″-O-dimethylerythromycin A 6,9-hemiacetal 11,12-carbonate. It thus appeared that the desired 6-O-methylerythromycin A 11,12-carbonate could not be prepared by methylation of erythromycin A 11,12-carbonate.

We therefore attempted to prepared 6-O-methylerythromycin A 11,12-carbonate by the reaction of ethylene carbonate with 6-O-methylerythromycin A using the previously described procedure, but that attempt met with failure.

In comparison, the corresponding 6-O-acetyl erythromycin 11,12-carbonate was prepared from erythromycin A 11,12-carbonate after initial protection of the more reactive hydroxyl groups (JP 62.142 194, Toyo Jozo, published 25.06.87).

A 2′-modified derivative, 2′-acetoxy-6-O-methyl erythromycin A 11,12-carbonate has been recently described in EP 0 248 279 (Baker et al, Abbott, filed 21.05.87, published 09.12.87, subsequent to the priority date of the present application). This compound was isolated as a by-product from the treatment of 2′-acetoxy-4″- benzyloxycarbonyl protected derivative of 6-O-methyl erythromycin A with carbonyl diimidazole, in the presence of sodium hexamethyldisilazide, the major product being the 12-acyl imidazole which was subsequently converted to the 11,12-carbamate by reaction with an amine.

We have now found that it is possible to prepare 11,12-carbonate derivatives of certain erythromycin 6-ethers by another method.

The present invention provides antibacterially active 11,12-carbonate derivatives of certain erythromycin 6-ether compounds.

In particular, the present invention provides a compound of general formula I or a pharmaceutically acceptable ester or acid addition salt thereof:

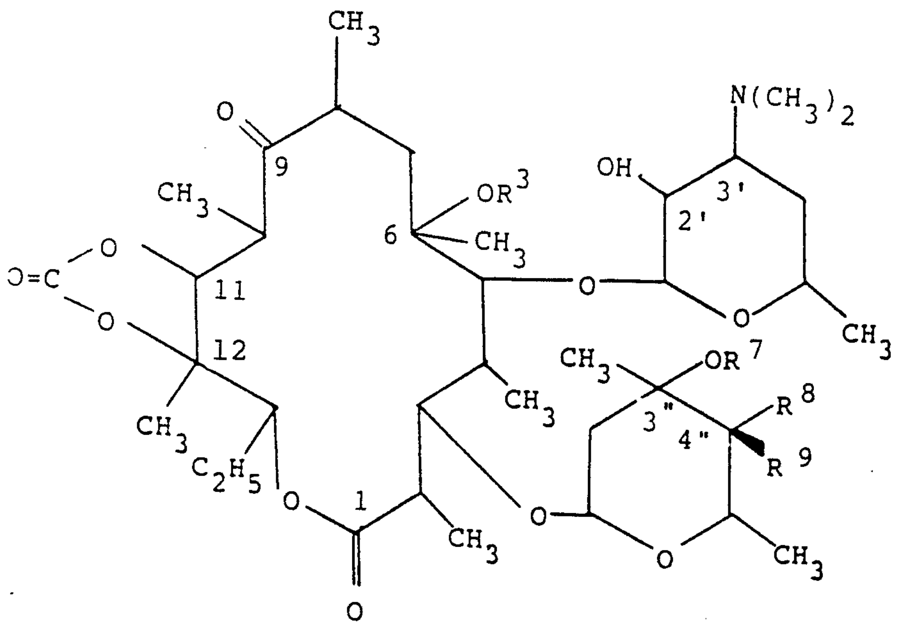

I

wherein

$R^3$ denotes a methyl or ethyl group;

$R^7$ denotes hydrogen or methyl;

one of $R^8$ and $R^9$ denotes hydrogen, hydroxy, alkoxy, alkanoyloxy, amino, substituted amino, or a group of the formula $R^A$-$SO_2$-O-, in which $R^A$ denotes an organic group, and the other of $R^8$ and $R^9$ denotes hydrogen, or

$R^8$ and $R^9$ together denote an oxo group, an oxime group, or a substituted oxime group.

The term 'hydrocarbon' as used herein includes groups having up to 18 carbon atoms, suitably up to 10 carbon atoms, conveniently up to 6 carbon atoms. Suitable hydrocarbon groups include $(C_{1-2})$alkyl, $(C_{2-6})$-alkenyl, $(C_{2-6})$alkynyl, $(C_{3-7})$cycloalkyl, aryl, $(C_{3-7})$cycloalkyl$(C_{1-6})$alkyl, aryl$(C_{1-6})$alkyl, $(C_{1-6})$alkyl$(C_{3-7})$-cycloalkyl, and $(C_{1-6})$alkylaryl.

Examples of suitable optional substituents for the above-mentioned hydrocarbon groups include heterocylyl, amino, $(C_{1-6})$alkanoylamino, (mono, di, or tri)-$(C_{1-6})$alkylamino, hydroxy, $(C_{1-6})$alkoxy, mercapto, $(C_{1-6})$alkylthio, heterocyclylthio, arylthio, sulphamoyl, carbamoyl, amidino, guanidino, nitro chloro, bromo, fluoro, carboxy and salts and esters thereof, $(C_{1-6})$alkanoyloxy, arylcarbonyloxy, heterocyclylcarbonyloxy and acyl groups.

Any alkyl group or moiety referred to herein may be straight or branched, unsubstituted or substituted, and may contain, for example, up to 12 carbon atoms, suitably up to 6 carbon atoms. In particular, the alkyl group or moiety may be an unsubstituted or substituted methyl, ethyl, n-propyl, iso-propyl, n-butyl, sec-butyl, isobutyl or tert-butyl group. Examples of suitable optional substitutents for any such alkyl group or moiety include the above-listed substitutents for hydrocarbon groups, and also the above-listed non-alkyl hydrocarbon groups, for example $(C_{2-6})$alkenyl and aryl groups.

The term 'aryl' as used herein includes phenyl and naphthyl, which may be unsubstituted or substituted by up to five, preferably up to three, groups selected from the above-listed substituents for hydrocarbon groups, and the above-listed hydrocarbon groups, including, for example, substituents selected from halogen, $(C_{1-6})$alkyl, phenyl, $(C_{1-6})$alkoxy, halo$(C_{1-6})$alkyl, hydroxy, amino, nitro, carboxy, $(C_{1-6})$-alkoxycarbonyl, $(C_{1-6})$alkoxycarbonyl$(C_{1-6})$alkyl, $(C_{1-6})$alkanoyloxy, and $(C_{1-6})$alkanoyl groups.

The terms 'acyl' as used herein includes formyl, unsubstituted and substituted hydrocarbon-carbonyl and hydrocarbonoxy-carbonyl groups, including, for example, unsubstituted and substituted alkanoyl, cycloalkycarbonyl, arylcarbonyl, alkoxycarbonyl, aryloxycarbonyl, and heterocyclylcarbonyl groups. The term 'acyloxy' is used analogously.

The terms 'heterocyclyl' and 'heterocyclic' as used herein include aromatic and non-aromatic, single and fused, rings suitably containing up to four hetero-atoms in each ring selected from oxygen, nitrogen and sulphur, which rings may be unsubstituted or substituted by, for example, up to three groups selected from halogen, $(C_{1-6})$alkyl, $(C_{1-6})$alkoxy, halo$(C_{1-6})$alkyl, hydroxy, amino, carboxy, carboxy salts, carboxy esters $(C_{1-6})$alkoxycarbonyl, $(C_{1-6})$alkoxycarbonyl$(C_{1-6})$alkyl, aryl, and oxo groups. Each heterocyclic ring suitably has from 4 to 7, preferably 5 or 6, ring atoms. A fused heterocyclic ring system may include

carbocyclic rings and need include only one heterocyclic ring.

The term 'heteroaryl' as used herein means an aromatic heterocyclic ring or ring system, suitably having 5 or 6 ring atoms in each ring.

The erythromycin derivatives according to the invention are characterised by a 11, 12-carbonate group, -O-CO-O-, and a 6-O-methyl or 6-O-ethyl group, -O-$R^3$.

Preferably, $R^3$ denotes a methyl group.

Various 6-O-methyl derivatives of erythromycin have been described in EP 0 041 355 A1, EP 0 080 818 A1, EP 0 080 819 A1, and EP 0 158 467 A2 (all Taisho Pharmaceutical). 6-O-ethyl derivatives may be prepared analogously.

The -O$R^7$ group in the 3″-position of the cladinose ring may be a hydroxy group or a methoxy group. Preferably, $R^7$ denotes a methyl group as in erythromycin A.

The 4″-position of the cladinose ring may suitably carry a hydroxy group as in erythromycin A ($R^8$ = H; $R^9$ = OH). Various modifications of the 4″-position of the cladinose ring have previously been described and those modifications may be incorporated in the compounds according to the present invention:

(i) 4″-deoxy-4″-oxo derivatives ($R^8$ + $R^9$ = O=) are described in US 3 842 069 and US 3 884 903, both P.H. Jones et al, Abbott Laboratories, and US 4 150 220, F.C. Sciavolino, Pfizer;

(ii) 4″-epi-hydroxy derivatives ($R^8$ = OH; $R^9$ = H) and 4″-deoxy-4″-alkanoyloxy-4″-epi derivatives ($R^8$ = alkanoyloxy, especially $CH_3COO$-; $R^9$ = H) are described in US 3 884 903, op. cit., and US 4 382 085, F.C. Sciavolino, Pfizer;

(iii) 4″-O-alkyl derivatives ($R^8$ or $R^9$ = alkoxy, especially methoxy; the other of $R^8$ and $R^9$ = H) are described in EP O 080 818 A1, op. cit.;

(iv) 4″-deoxy-4″-amino derivatives ($R^8$ or $R^9$ = amino or substituted amino; the other of $R^8$ and $R^9$ = H) are described in US 4 150 220, op. cit.;

(v) 4″-deoxy-4″-oxime derivatives ($R^8$ + $R^9$ = oxime (=N-OH) or substituted oxime, especially acetyloxime (=N-O-CO-$CH_3$)) are also described in US 4 150 220, op cit.;

(vi) 4″-O-sulphonyl derivatives ($R^8$ = H, $R^9$ = $R^A$-$SO_2$-O-) are described in US 3 836 519, US 3 869 445 and US 4 063 014, all R. Hallas et al, Abbott Laboratories;

(vii) 4″-deoxy derivatives ($R^8$ = $R^9$ = H) are described in JP 58-049396, Toyo Jozo KK.

In the 4″-deoxy-4″-(substituted amino) derivatives, the substituted amino group $R^8$ or $R^9$ may suitably be a group of the formula II or III:

-NHCO$R^F$ II or -NH$SO_2R^F$ III

in which $R^F$ denotes a hydrocarbon group.

In the 4″-O-sulphonyl derivatives, in which $R^8$ or $R^9$ denotes a sulphonyloxy group of the formula IV:

$R^A$-$SO_2$-O-, IV

the organic group $R^A$ may suitably be an unsubstituted or substituted hydrocarbon, oxahydrocarbon, thiahydrocarbon or azahydrocarbon group, more especially an alkyl, alkenyl, unsubstituted or substituted aryl (especially phenyl, nitrophenyl, halophenyl or alkylphenyl), unsubstituted or substituted aralkyl (especially benzyl, nitrobenzyl, halobenzyl or alkylbenzyl), unsubstituted or substituted aryloxyalkyl (especially phenoxyalkyl, nitrophenoxyalkyl, halophenoxyalkyl or alkylphenoxyalkyl), or substituted ethyl (especially $R^G$-$CH_2$-$CH_2$-, wherein $R^G$ is defined as below) group.

Examples of group $R^G$ in the 4″-substituent

$R^G$-$CH_2$-$CH_2$-$SO_2$-O- V

include amino, substituted amino, carbamoyl, substituted carbamoyl, sulphamoyl, substituted sulphamoyl, substituted ureido, substituted thioureido, alkoxy, alkythio, optionally substituted aryloxy, optionally substituted arylthio, optionally substituted benzyloxy, optionally substituted benzylthio, substituted sulphonyl, substituted sulphinyl, substituted alkyl, substituted alkanoyl, substituted cyano, and other groups more specifically described in US 3 869 445 and US 4 063 014, op. cit.

Preferably, $R^A$ denotes a hydrocarbon group, particularly a ($C_{1-6}$)alkyl group, especially a methyl group.

The present invention includes pharmaceutically acceptable esters, especially in vivo hydrolysable esters, of the compounds of the general formula I. Such esters may be formed at any hydroxy group in the

compounds of the general formula I, but usually the ester will be formed at the 2′-hydroxy group of the desosamine ring, thus giving a 2′-hydroxy group of the desosamine ring, thus giving a 2′-O-acyl derivative of the type described in US 2 862 921 (R.E. Booth et al; Upjohn Co.), US 2 993 883 (V.C. Stephens; Eli Lilly), US 3 884 904 (P.H. Jones et al, Abbot Laboratories), US 3 836 519, US 3 842 069, US 3 869 445, US 3 884 903, and US 4 150 220, all op. cit..

Suitable pharmaceutically acceptable in vivo hydrolysable esters include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic, and alkanedioic acids, in which each alkyl or alkenyl moiety advantageously has not more than 6 carbon atoms. Examples of particular esters include formates, acetates, propionates, butyrates, acrylates, and ethylsuccinates.

The present invention also includes acid addition salts, especially pharmaceutically acceptable acid addition salts, of the compounds of the general formula I. Such acid addition salts may, in particular, be formed at the 3′-dimethylamino group of the desosamine ring.

Various acid addition salts of erythromycin are described in US 2 761 859 (C.E. Hoffhine, Jr.; Abbott Laboratories) and US 2 852 429 (J.T. Shepler; Eli Lilly).

Suitable acid addition salts of the compounds of the invention include pharmaceutically acceptable inorganic acid addition salts, for example the sulphate, nitrate, phosphate, borate, hydrochloride and hydrobromide, and also pharmaceutically acceptable organic acid addition salts, for example the acetate, tartrate, maleate, citrate, succinate, benzoate, ascorbate, methane-sulphate, α-keto-glutarate, α-glycerophosphate, and glucose-1-phosphate. Preferably the acid addition salt is the laurylsulphate salt.

Examples of individual compounds according to the present invention include

6-O-methyl-erythromycin A 11,12-carbonate (in general formula I, $R^3$ = $CH_3$; $R^7$ = $CH_3$; $R^8$ = H; $R^9$ = OH;

as well as

corresponding derivatives in which the 4″-position is modified as discussed above;

and also

pharmaceutically acceptable esters and acid addition salts of such compounds.

The erythromycin 6-ether 11,12-carbonate derivatives according to the invention may be prepared by reacting an erythromycin A 6-O-methyl or 6-O-ethyl derivative having free hydroxy substituents at the 11- and 12-positions, in which any reactive groups (other than the 11- and 12-hydroxy groups) may optionally be protected, with a reactive carbonyl compound; and thereafter if necessary carrying out one or more of the following steps

(a) converting a substituent on the erythromycin structure to another such substituent in a conventional manner;

(b) removing any protecting groups; and

(c) forming a pharmaceutically acceptable ester or acid addition salt.

More particularly, a compound of the general formula I as hereinbefore defined or a pharmaceutically acceptable ester or acid addition salt thereof may be prepared by a process which comprises reacting a compound of the general formula VI:

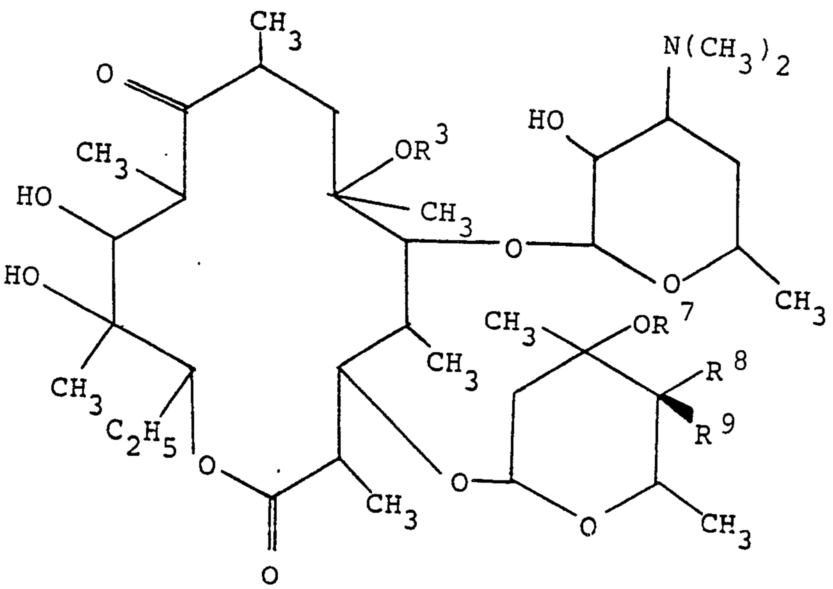

VI

wherein
$R^3$, $R^7$, $R^8$ and $R^9$ are defined as above with respect to general formula I, in which compound of the general formula VI any reactive group (other than the 11- and 12-hydroxy groups) may optionally be protected, with a reactive carbonyl compound,
to give a compound of the general formula I,
and thereafter, if necessary or desired, carrying out one or more of the following steps in any suitable order:

(a) converting any one or more of the groups denoted by $R^3$, $R^8$ and $R^9$ to another such group;
(b) removing any protecting group that may be present; and
(c) forming a pharmaceutically acceptable ester or acid addition salt.

The compound of the general formula VI in which:
each of $R^3$ and $R^7$ denotes methyl,
$R^8$ denotes hydrogen, and
$R^9$ denotes hydroxy,
is 6-O-methylerythromycin A, which may be prepared from erythromycin A by known methods, for example by the methods described in the above-cited references relating to 6-O-methylerythromycins

Compounds of the general formula VI in which $R^3$ denotes an ethyl group may be prepared by analogous methods.

Other compounds of the general formula VI may also be prepared, by methods known per se, from erythromycin A or a corresponding 6-O-methyl or ethyl derivative. For example, a compound in which the 4″-position is substituted other than as in naturally-occurring erythromycin A (that is to say, in which $R^8$ is other than hydrogen and/or $R^9$ is other than hydroxy) may be prepared as described in the respective references cited above.

Prior to carrying out the reaction of a compound of the general formula VI with the reactive carbonyl compound, any reactive group of a compound of the general formula VI (other than the 11- and 12-hydroxy group) may optionally be protected.

For example, it has been found advantageous to protect the 3′-dimethylamino group by means of an N-protecting group is known manner, for example by the method described by E.H. Flynn et al, (J. Amer. Chem. Soc., 1955, 77, 3104-3106.

Examples of suitable N-protecting groups include benzyloxycarbonyl, and substituted benzyloxycarbonyl, (for example, p-methylbenzyloxycarbonyl, p-nitrobenzyloxycarbonyl, p-bromobenzyloxycarbonyl, p-phenylazobenzyloxycarbonyl, and p-(p′-methoxyphenylazo)-benzyloxycarbonyl). A preferred N-protecting group is benzyloxycarbonyl.

It may also be advantageous to protect one or more of the hydroxy groups present in the erythromycin molecule (other than the 11- and 12-hydroxy groups) prior to the reaction. In particular, it may be advantageous to protect the hydroxy group present at the 2′-position. It is convenient to employ the same group to protect the 2′-hydroxy group as that employed to protect the amino moiety, especially a benzyloxycarbonyl group.

Any reactive substituents that may be present in the group $R^8$ or $R^9$ should preferably also be protected in a conventional manner.

In the process according to the invention, the erythromycin compound of the general formula VI, optionally containing protective groups, is reacted with a reactive carbonyl compound, which may be phosgene $COCl_2$ or oxalyl chloride $(COCl)_2$.

The reactive carbonyl compound may suitably be used in an excess of from 1 to 10 equivalents, based on the erythromycin compound.

The reaction may conventionally be carried out at a temperature within the range of from -50 to +50° C, preferably from -20 to +30° C, in an inert solvent and, preferably, in the presence of a weak base (e.g. pyridine) as an acid acceptor.

Suitable inert solvents for the reaction include, for example, dichloromethane, chloroform, diethyl ether, tetrahydrofuran, dioxane, and dimethoxyethane (although the lower boiling solvents will not, of course, be suitable in cases where the reaction is carried out at higher temperatures).

After completion of the reaction with the carbonyl compound, the group(s) $R^8$ and $R^9$ may be converted to any of the other such groups within the definitions given above by methods known in the art, for example by the methods disclosed in the above-cited reference. For example, a compound in which $R^9$ denotes hydrogen and $R^8$ denotes hydroxy can be converted to a compound in which $R^8$ and $R^9$ together denote oxo and optionally thereafter to a compound in which $R^9$ denotes hydroxy or acetoxy and $R^8$ denotes hydrogen by methods analogous to those described in US 3 884 903, op. cit..

After completion of the reaction with the carbonyl compound, any protecting groups (such as a benzyloxycarbonyl group) may be removed by a conventional method. It is often appropriate to employ a hydrogenation procedure.

The hydrogenation may suitably be carried out in the presence of a transition metal catalyst, for example palladium, which may, for example, be in the form of palladium on carbon (charcoal), palladium on barium sulphate, palladium on calcium carbonate, or palladium black. A favoured catalyst is palladium on carbon (sometimes referred to as palladium on charcoal); for example 5%, 10%, 20% or 30% palladium on carbon. A low, medium or high pressure of hydrogen may be used in this reaction, for example a pressure of from 1 to 6 atmospheres absolute, a pressure of 1 atmosphere absolute being convenient. The reaction may suitably be carried out at a non-extreme temperature, for example at a temperature within the range of from 0° C to 30° C, preferably from 12° C to 25° C. It is generally convenient to carry out the reaction at ambient temperature. The reaction is preferably carried out at a pH within the range of from 4.5 to 5.0, which may be maintained by the use of a suitable buffer, for example an acetate buffer at pH 4.8. Suitable solvents for carrying out the hydrogenation include ethanol, n-propanol, isopropanol, tetrahydrofuran, dioxan, ethyl acetate, a mixture of two or more such solvents, or such a solvent or mixture in the presence of water. A favoured solvent is ethanol.

When necessary, the dimethylamino group at the 3′-position may conventionally be restored by effecting a reductive methylation, which advantgeously may be carried out at the same time as the reductive removal of the protecting groups, as in the method of Flynn et al, op. cit..

A compound of the general formula I may be converted to a pharmaceutically acceptable salt thereof of ester thereof in a conventional manner at any convenient stage in the manufacturing process, for example before or after the removal of any protecting groups and/or before or after any conversion of groups $R^8$ and $R^9$ to other such groups.

Isolation and purification of a compound according to the invention may be carried out using conventional methods, and may include a chromatography step. Preferably the product is isolated in crystalline form.

The compounds according to the invention, that is to say, the compounds of the general formula I and their pharmaceutically acceptable salt and esters, have antibacterial properties and are useful for the treatment of bacterial infections in animals, especially mammals, including humans, in particular humans and domesticated animals (including farm animals). The compounds may be used for the treatment of infections caused by a wide range of gram-positive and gram-negative organisms including, for example, Bacillus subtilis, Corynebacterium xerosis, Sarcina lutea, Staphylococcus aureus, Streptococcus faecalis, Streptococcus pyogenes, Streptococcus agalactiae, Streptococcus pneumoniae, Haemophilis sp. Neisseria sp., Chlamydia sp., and Legionella sp..

The present invention provides a pharmaceutical composition comprising a compound according to the invention together with a pharmaceutically acceptable carrier or excipient.

The present invention also provides a method of treating bacterial infections in animals, especially in humans and in domesticated mammals, which comprises administering a compound or composition according to the invention to a patient in need thereof.

The compounds and compositions according to the invention may be formulated for administration in any convenient way for use in human or veterinary medicine, by analogy with other antibiotics.

The compounds and compositions according to the invention may be formulated for administration by any route, for example oral, topical or parenteral. The compositions may, for example, be made up in the form of tablets, capsules, powders, granules, lozenges, creams, syrups, or liquid preparations, for example solutions or suspensions, which may be formulated for oral use or in sterile form for parenteral administration by injection or infusion.

Tablets and capsules for oral administration may be in unit dosage form, and may contain conventional excipients including, for example, binding agents, for example syrup, acacia, gelatin, sorbitol, tragacanth, or polyvinylpyrollidone; fillers, for example lactose, sugar, maize-starch, calcium phosphate, sorbitol or glycine; tabletting lubricants, for example magnesium stearate, talc, polyethylene glycol or silica; disintegrants, for example potato starch; and pharmaceutically acceptable wetting agents, for example sodium lauryl sulphate. The tablets may be coated according to methods well known in normal pharmaceutical practice.

Oral liquid preparations may be in the form of, for example, aqueous or oily suspensions, solutions, emulsions, syrups or elixirs, or may be presented as a dry product for reconsititution with water or another suitable vehicle before use. Such liquid preparations may contain conventional additives, including, for example, suspending agents, for example sorbitol, methyl cellulose, glucose syrup, gelatin, hydroxyethyl cellulose, carboxymethyl cellulose, aluminium stearate gel or hydrogenated edible fats; emulsifying agents, for example lecithin, sorbitan monooleate or acacia; non-aqueous vehicles (which may include edible oils), for example almond oil, oily esters (for example glycerine), propylene glycol, or ethyl alcohol; preservatives, for example methyl or propyl p-hydroxybenzoate or sorbic acid; and, if desired, conventional flavouring and colouring agents.

A compound or composition according to the invention may suitably be administered to the patient in an antibacterially effective amount.

A composition according to the invention may suitably contain from 0.1% by weight, preferably from 10 to 60% by weight, of a compound according to the invention (based on the total weight of the composition), depending on the method of administration.

The compounds according to the invention may suitably be administered to the patient at a daily dosage of from 1.5 to 50 mg/kg of body weight. For an adult human (of approximately 70 kg body weight), from 100 to 3000 mg, for example about 1500 mg, of a compound according to the invention may be administered daily. Suitably, the dosage for adult humans is from 5 to 20 mg/kg per day. Higher or lower dosages may, however, be used in accordance with normal clinical practice.

When the compositions according to the invention are presented in unit dosage form, each unit dose may suitably comprise from 25 to 1000 mg, preferably from 50 to 500 mg, of a compound according to the invention.

No adverse toxicological effects are indicated when the compounds according to the invention are administered within the above-mentioned dosage ranges.

The following examples illustrate the preparation of compounds according to the present invention. The percentage yields quoted throughout the examples are calculated on the theoretical yield from the corresponding erythromycin derivative used as the respective starting material.

Example 1

6-O-Methylerythromycin A 11,12-carbonate (5)

2′-O,3′-N-Bis(benzyloxycarbonyl)-N-desmethyl-6-O-methylerythromycin A (1) (EP 0 147 062, Example 1, Taisho) (3.5 g) in tetrahydrofuran (100ml) was stirred with anhydrous potassium carbonate (2.5g) and carbonyl di-imidazole (0.71g) for 1h. After evaporating the solvent the residue was dissolved in ethyl acetate, washing with water, then dilute citric acid, finally dried (MgSO4) and evaporated. The residue was chromatographed on silica gel eluting with ethyl acetate/hexane (1/1) to give 4″-O-(N-imidazoylcarbonyl)-2′-O,3′-N-bis(benzyloxycarbonyl)-N-desmethyl-6-O-methylerythromycin A (2) (1.39g, 36%) as an amorphous solid.

A mixture of compound (2) (1.13g) and benzyl alcohol (1ml) in tetrahydrofuran (20ml) was stirred at 20°C with 50% sodium hydride suspension (10mg) for 30min and the product was purified by chromatography eluting with dichloromethane/ethyl acetate mixtures to yield 2′-O,3′-N,4″-O-tris(benzyloxycarbonyl)-N-desmethyl 6-O-methylerythromycin A (3) (473mg, 40%).

Compound (3) (370mg) in dichloromethane (40ml) and pyridine (3.5ml) at 20° C was treated with a 12.5% solution of phosgene in toluene (3.5ml). After 1h aqueous sodium hydrogen carbonate was added and the mixture was evaporated to low bulk in vacuo, then the residue was dissolved in ethyl acetate, washed with dilute citric acid, dried ($MgSO_4$) and evaporated. The product was purified by chromatography on silica gel, eluting with ethyl acetate/dichloromethane (15/85).

This gave 2′-O,3′-N,4″-O-tris(benzyloxycarbonyl)-N-desmethyl-6-O-methylerythromycin A 11,12-carbonate (4) (214mg, 56%).

The above compound (4) (100mg) in ethanol (15ml) and pH 4.8 acetate buffer (1.5ml) was hydrogenated over 10% Pd on carbon (20mg) for 1h at 20° C. Aqueous formaldehyde solution (38%, 0.1ml) was added and hydrogenation was resumed for a further 1h. The mixture was filtered and concentrated then partitioned between ethyl acetate and aqueous sodium hydrogen carbonate. The solvent layer was dried ($MgSO_4$) and evaporated to yield the title compound (5) (57mg, 86%) as a white solid, $\nu_{max}$ ($CHCl_3$) 1800, 1730 $cm^{-1}$ $[\alpha]^{20}D$ (1%, $CHCl_3$) -47.0°, FAB m.s. 796 $MNa^+$.

## Example 2

6-O-Methylerythromycin A 11,12-carbonate (5)

To a solution of 2′-O,3′-N-bis(benzyloxycarbonyl)-N-desmethyl-6-O-methylerythromycin A (1) (1.0g) in dichloro- methane (100ml) and pyridine (4ml) at 20° C was added a solution of phosgene (12.5% w/v in toluene) portionwise over 3h until a total of 8ml had been added. (By this time t.l.c. indicated all the starting material to have been consumed). The reaction mixture was then washed with aqueous sodium hydrogen carbonate, water, and dilute citric acid, and then dried ($MgSO_4$) and evaporated. The residue was chromatographed on silica gel eluting with dichloromethane/ethyl acetate (4/1), the main fraction being 2′-O,3′-N-bis(benzyloxycarbonyl)-N-desmethyl-6-O-methylerythromycin A 11,12-carbonate (6), a white amorphous solid (565mg, 55%) $\nu_{max}$ ($CHCl_3$) 1800, 1730, 1690 $cm^{-1}$. Found: m.s. FAB $MNa^+$ 1050. The above product (797mg) in ethanol (50ml) and pH 4.8 acetate buffer (5ml) was then hydrogenated as in Example 1 and the product was purified by chromatography on silica gel eluting with dichloromethane/methanol/0.880 ammonia (100/10/1) to give the title compound (430mg, 72%) identical with the product of Example 1.

## Claims

1. A compound of the general formula I or a pharmaceutically acceptable ester or acid addition salt thereof:

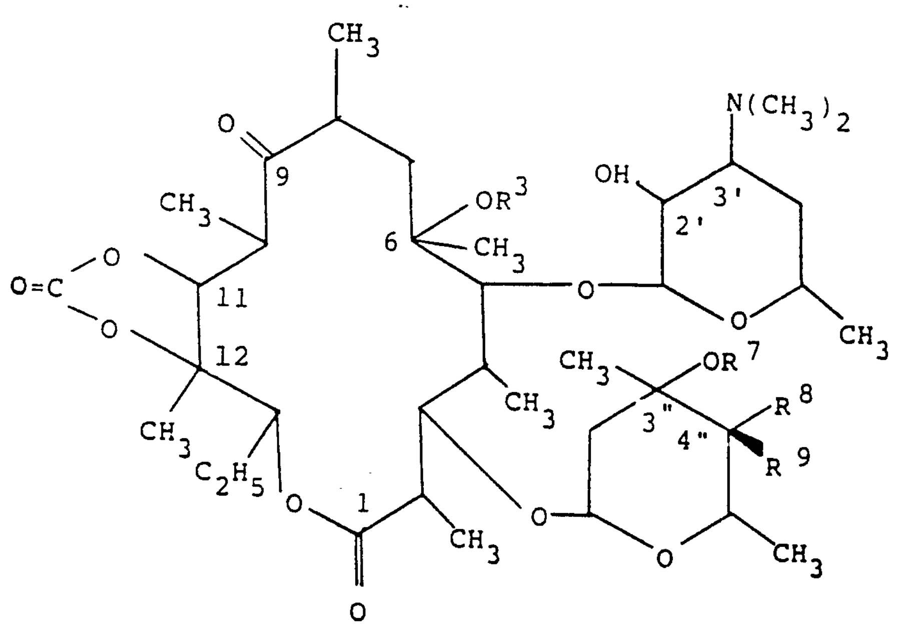

I

wherein
$R^3$ denotes a methyl or ethyl group;
$R^7$ denotes hydrogen or methyl;
one of $R^8$ and $R^9$ denotes hydrogen, hydroxy, alkoxy, alkanoyloxy, amino, substituted amino, or a group of the formula $R^A$-$SO_2$-O-, in which $R^A$ denotes an organic group, and the other of $R^8$ and $R^9$ denotes hydrogen, or
$R^8$ and $R^9$ together denote an oxo group, an oxime group, or a substituted oxime group.

2. A compound as claimed in claim 1, wherein $R^3$ denotes a methyl group.

3. A compound as claimed in claim 1 or claim 2, wherein $R^7$ denotes a methyl group.

4. A compound as claimed in any one of claims 1 to 3, wherein $R^8$ denotes hydrogen and $R^9$ denotes hydroxyl.

5. 6-O-methyl-erythromycin A 11,12-carbonate and pharmaceutically acceptable esters and acid addition salts thereof.

6. A process for the preparation of a compound of the general formula I as given in claim 1 or a pharmaceutically acceptable ester or acid addition salt thereof which comprises reacting a compound of the general formula VI:

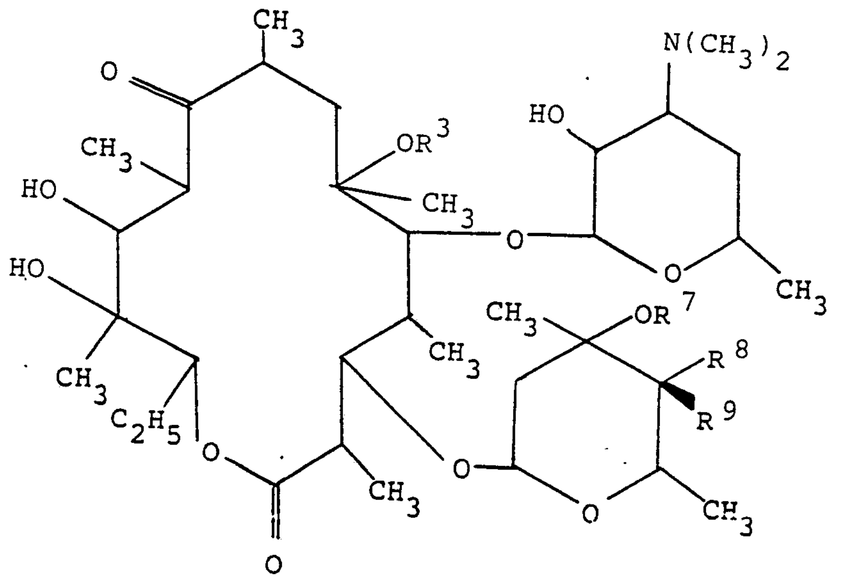

VI

wherein
$R^3$, $R^7$, $R^8$ and $R^9$ are as defined in claim 1, and in which compound of the general formula VI any reactive group (other than the 11- and 12-hydroxy groups) may optionally be protected,
with a reactive carbonyl compound,
to give a compound of the general formula I,
and thereafter, if necessary or desired, carrying out one or more of the following steps in any suitable order:
(a) converting any one or more of the groups denoted by $R^3$, $R^8$ and $R^9$ to another such group;
(b) removing any protecting group that may be present; and
(c) forming a pharmaceutically acceptable ester or acid addition salt.

7. A pharmaceutical composition comprising a compound as claimed in any one of claims 1 to 5 together with a pharmaceutically acceptable carrier or excipient.

8. A compound as claimed in any one of claims 1 to 5 for use in the treatment of bacterial infections.

9. The use of a compound as claimed in any one of claims 1 to 5 in the manufacture of a medicament.